# EUROPEAN PATENT APPLICATION

(11) **EP 2 144 056 A1**
(43) Date of publication of application: **13.01.2010**
(21) Application number: 08751964.1
(22) Date of filing: 23.04.2008
(51) Int. Cl.: G01N 27/447, G01N 37/00

(54) **ELECTROPHORESIS CHIP AND ELECTROPHORESIS APPARATUS**

(30) Priority: 27.04.2007 JP 2007119260
(71) Applicant: Arkray, Inc., Kyoto-shi, Kyoto 601-8045 (JP)
(72) Inventor: SUGIYAMA, Koji, Kyoto 601-8045 (JP); YONEHARA, Satoshi, Kyoto 601-8045 (JP); NAKAYAMA, Yusuke, Kyoto 601-8045 (JP)
(74) Representative: Behnisch, Werner
(86) International application number: PCT/JP2008/057826
(87) International publication number: WO 2008/139866

(57) **Abstract**

An electrophoresis chip that enables an apparatus to be small, analysis time to be short and glycosylated hemoglobin to be analyzed highly accurately is provided. The electrophoresis chip includes an upper substrate 4, a lower substrate 1, a first introduction reservoir 2a, a first recovery reservoir 2b and a capillary channel for sample analysis 3x; the first introduction reservoir 2a and the first recovery reservoir 2b are formed in the lower substrate 1; and the first introduction reservoir 2a and the first recovery reservoir 2b are in communication with each other via the capillary channel for sample analysis 3x.

## Description

### Technical Field

The present invention relates to an electrophoresis chip and an electrophoresis apparatus.

### Background Art

The degree of glycosylation of various proteins has been analyzed as an indicator that shows the condition of a living body. In particular, since the degree of glycosylation of hemoglobin (Hb), especially HbA1c, in blood cells reflects the history of glucose levels in a living body, it is regarded as an important indicator in the diagnosis, treatment or the like of diabetes.
HbA1c is HbA(α₂β₂) whose β-chain N-terminal valine has been glycosylated.

HbA1c has been analyzed by, for example, immunological methods, enzymatic methods, high-performance liquid chromatography (HPLC) methods, and affinity methods, among othes. Although immunological methods and enzymatic methods are generally used in processing and analyzing large numbers of specimens, they are of low accuracy when determining the risks due to complications. On the other hand, although HPLC methods have a poorer processing capability than immunological methods or enzymatic methods, they are useful in determining the risk of complications. However, due to the configuration of HPLC methods, the analysis apparatus is very large and costly. In affinity methods, types of glycosylated Hb other than HbA1c whose β-chain N-terminal have been glycosylated are also measured concurrently.

Furthermore, analysis of HbA1c using capillary electrophoresis has been attempted (see Non-Patent Document 1). This method, however, uses a fused silica capillary having an inner diameter of 25 µm, which is not advantageous in terms of sensitivity, and requires an analysis time of about 4 minutes for the analysis of HbA1c. Moreover, this method requires the use of a large electrophoresis apparatus. In addition, POC (point of care) testing using any of the aforementioned conventional methods is not sufficiently accurate to enable the management of the risks due to complications, and it has been used as no more than a screening test. These problems can be associated with glycosylated hemoglobin, including HbA1c, as a whole.

Non-Patent Document 1: Clinical Chemistry 43: 4, 644-648 (1997)

### Disclosure of Invention

Therefore, an object of the present invention is to provide electrophoresis chips, for the analysis of glycosylated hemoglobin by capillary electrophoresis, that allows an apparatus to be small, analysis time to be short and glycosylated hemoglobin to be analyzed highly accurately.

To achieve the above object, electrophoresis chips of the present invention are electrophoresis chips for analyzing glycosylated hemoglobin; electrophoresis chips include a substrate, a plurality of fluid reservoirs and a capillary channel;
the plurality of fluid reservoirs include a first introduction reservoir and a first recovery reservoir;
the capillary channel includes a capillary channel for sample analysis;
the first introduction reservoir and the first recovery reservoir are formed in the substrate; and
the first introduction reservoir and the first recovery reservoir are in communication with each other via the capillary channel for sample analysis.

Electrophoresis apparatuses of the present invention are electrophoresis apparatuses that includes an electrophoresis chip and an analysis unit, with the electrophoresis chip being an electrophoresis chip of the present invention.

Electrophoresis chips of the present invention are chips wherein a first introduction reservoir and a first recovery reservoir are formed in a substrate, and the first introduction reservoir and the first recovery reservoir are in communication with each other via a capillary channel for sample analysis. Hence, in an analysis of glycosylated hemoglobin by capillary electrophoresis, the present invention allows an apparatus to be small and accordingly an analysis time to be short. Moreover, it is possible with electrophoresis chips of the present invention to analyze glycosylated hemoglobin highly accurately. Therefore, it is possible with electrophoresis chips of the present invention to accurately analyze glycosylated hemoglobin in, for example, POC testing, and thus, to manage the risks due to complications.

### Brief Description of Drawings

[FIG. 1] FIG. 1 shows diagrams illustrating a configuration of an example of an electrophoresis chip of the present invention.
[FIG. 2] FIG. 2 is a flowchart illustrating an example of a production process of an electrophoresis chip of the present invention.
[FIG. 3] FIG. 3 is a flowchart illustrating another example of a production process of an electrophoresis chip of the present invention.
[FIG. 4] FIG. 4 shows diagrams illustrating a configuration of another example of an electrophoresis chip of the present invention.
[FIG. 5] FIG. 5 shows diagrams illustrating a configuration of an example of an electrophoresis apparatus of the present invention.
[FIG. 6] FIG. 6 shows diagrams illustrating a configuration of another example of an electrophoresis apparatus of the present invention.
[FIG. 7] FIG. 7 shows diagrams illustrating a configuration of still another example of an electrophoresis chip of the present invention.
[FIG. 8] FIG. 8 shows diagrams illustrating a configuration of still another example of an electrophoresis chip of the present invention.
[FIG. 9] FIG. 9 shows diagrams illustrating a configuration of still another example of an electrophoresis chip of the present invention.
[FIG. 10] FIG. 10 shows diagrams illustrating a configuration of still another example of an electrophoresis chip of the present invention.
[FIG. 11] FIG. 11 shows diagrams illustrating a configuration of still another example of an electrophoresis apparatus of the present invention.
[FIG. 12] FIG. 12 shows diagrams illustrating a configuration of still another example of an electrophoresis chip of the present invention.
[FIG. 13] FIG. 13 is a graph showing the relationship between the migration distance and the absorbance in an Example of the present invention.

### Description of the Preferred Embodiments

An electrophoresis chip of the present invention may be configured such that:
the plurality of fluid reservoirs further include a second introduction reservoir and a second recovery reservoir,
the capillary channel further includes a capillary channel for sample introduction,
the second introduction reservoir and the second recovery reservoir are formed in the substrate,
the second introduction reservoir and the second recovery reservoir are in communication with each other via the capillary channel for sample introduction,
the capillary channel for sample analysis and the capillary channel for sample introduction intersect, and
the capillary channel for sample analysis and the capillary channel for sample introduction are in communication with each other at the intersection.

An electrophoresis chip of the present invention may be configured such that:
a first branching channel branches off from a part of the capillary channel for sample analysis,
the first branching channel is in communication with the second introduction reservoir,
a second branching channel branches off from a part of the capillary channel for sample analysis that is located on the downstream side relative to the first branching channel,
the second branching channel is in communication with the second recovery reservoir, and
the capillary channel for sample introduction is formed by the first branching channel, the second branching channel and the part of the capillary channel for sample analysis that connects the branching channels.

In an electrophoresis chip of the present invention, the maximum length of the whole chip is in a range of, for example, 10 to 100 mm and preferably in a range of 30 to 70 mm; the maximum width of the whole chip is in a range of, for example, 10 to 60 mm; and the maximum thickness of the whole chip is in a range of, for example, 0.3 to 5 mm. The maximum length of the whole chip refers to the dimension of the longest portion of the chip in the longitudinal direction; the maximum width of the whole chip refers to the dimension of the longest portion of the chip in a direction (width direction) perpendicular to the longitudinal direction; and the maximum thickness of the whole chip refers to the dimension of the longest portion of the chip in a direction (thickness direction) perpendicular to both the longitudinal direction and the width direction.

It is preferable that an electrophoresis chip of the present invention is such that, in analyzing glycosylated hemoglobin, a diluted sample in which a sample containing glycosylated hemoglobin is diluted with an electrophoresis running buffer is introduced into at least one fluid reservoir of the plurality of fluid reservoirs, and the volume ratio of the sample : the electrophoresis running buffer is in a range of 1:4 to 1:99. The volume ratio of the sample : the electrophoresis running buffer is more preferably in a range of 1:9 to 1:59 and still more preferably in a range of 1:19 to 1:29.

In an electrophoresis chip of the present invention, it is preferable that the capillary channel is filled with an electrophoresis running buffer.

In an electrophoresis chip of the present invention, the maximum diameter of the capillary channel is in a range of, for example, 10 to 200 µm and preferably in a range of 25 to 100 µm; and the maximum length thereof is in a range of, for example, 0.5 to 15 cm. When the shape of the cross section of the capillary channel is not circular, the maximum diameter of the capillary channel refers to the diameter of a circle having an area that corresponds to the cross sectional area of a portion having the largest cross-sectional area.

In an electrophoresis chip of the present invention, the inner wall of the capillary channel may be coated with a cationic group-containing compound. Examples of the cationic group-containing compound include compounds that contain cationic groups and reactive groups. Preferable examples of the cationic groups include amino groups and ammonium groups. A preferable example of the cationic group-containing compound is a silylating agent that contains at least an amino group or an ammonium group. The amino groups may be any of the primary, secondary and tertiary amino groups.

Examples of the silylating agent include
N-(2-diaminoethyl)-3-propyltrimethoxysilane, aminophenoxydimethylvinylsilane, 3-aminopropyldiisopropylethoxysilane, 3-aminopropylmethylbis(trimethylsiloxy)silane, 3-aminopropylpentamethyldisiloxane, 3-aminopropylsilanetriol, bis(p-aminophenoxy)dimethylsilane, 1,3-bis(3-aminopropyl)tetramethyldisiloxane, bis(dimethylamino)dimethylsilane, bis(dimethylamino) vinylmethylsilane, bis(2-hydroxyethyl)-3-aminopropyltriethoxysilane, 3-cyanopropyl(diisopropyl)dimethylaminosilane, (aminoethylaminomethyl)phenethyltrimethoxysilane, N-methylaminopropyltriethoxysilane, tetrakis(diethylamino)silane, tris(dimethylamino)chlorosilane, and tris(dimethylamino)silane, among others.

Among such silylating agents, those in which silicon atom(s) are substituted with titanium or zirconium may be used. Such silylating agents may be used singly or may be used in a combination of two or more.

Coating of the inner wall of the capillary channel with the silylating agent is performed, for example, as follows. First, a silylating agent is dissolved or dispersed in an organic solvent to prepare a treatment fluid. Examples of the organic solvent for use in the preparation of the treatment fluid may be dichloromethane, toluene and the like. The concentration of the silylating agent in the treatment fluid is not particularly limited. This treatment fluid is passed through the capillary channel, and then heated. Due to this heating, the silylating agent is bonded to the inner wall of the capillary channel by covalent bonding, resulting in a cationic group being disposed on the inner wall of the capillary channel. Thereafter, washing (after-treatment) is performed with at least an organic solvent (dichloromethane, methanol, acetone or the like), an acid solution (phosphoric acid or the like), an alkaline solution or a surfactant solution. Although this washing is optional, it is preferable to perform such washing. Moreover, as described below, when a capillary tube that is a member independent of the substrate serves as the capillary channel, a capillary tube whose inner wall is coated with a cationic group-containing compound through the use of a commercially available silylating agent of an aforementioned kind may be used.

It is preferable that an anionic layer formed from an anionic group-containing compound is further coated on the inner wall of the capillary channel that has been coated with a cationic group-containing compound. It is thus possible to prevent hemoglobin or the like present in a sample that will be described below from being adsorbed onto the inner wall of the capillary channel. Moreover, due to the formation of a complex between the sample and the anionic group-containing compound and due to the electrophoresis thereof, separation efficiency is enhanced compared with the electrophoresis of a sample alone. As a result of these, analysis of glycosylated hemoglobin or the like can be performed more accurately in a shorter period of time. An anionic group-containing polysaccharide is preferable as the anionic group-containing compound that forms a complex with the sample. Examples of anionic group-containing polysaccharides include sulfated polysaccharides, carboxylated polysaccharides, sulfonated polysaccharides and phosphorylated polysaccharides. Among these, sulfated polysaccharides and carboxylated polysaccharides are preferable. The sulfated polysaccharides are preferably chondroitin sulfate, or heparin, among others, with chondroitin sulfate being particularly preferable. The carboxylated polysaccharides are preferably alginic acid and salts thereof (for example, sodium alginate). There are seven types of chondroitin sulfate, for example, chondroitin sulfate A, chondroitin sulfate B, chondroitin sulfate C, chondroitin sulfate D, chondroitin sulfate E, chondroitin sulfate H, and chondroitin sulfate K, and any of these types may be used. The anionic layer can be formed by, for example, bringing a fluid that contains an anionic group-containing compound into contact with the inner wall of the capillary channel that has been coated with a cationic group-containing compound. In this case, although a fluid for forming an anionic layer may be prepared separately, it is preferable in terms of operation efficiency that an electrophoresis running buffer that contains an anionic group-containing compound is prepared and is passed through the capillary channel whose inner wall is coated with a cationic group-containing compound.

The electrophoresis running buffer is not particularly limited, and an electrophoresis running buffer that uses an organic acid is preferable. Examples of organic acids include maleic acid, tartaric acid, succinic acid, fumaric acid, phthalic acid, malonic acid, and malic acid, among others. Preferably, the electrophoresis running buffer contains a weak base. Examples of weak bases include arginine, lysine, histidine, and tris, among others. The pH of the electrophoresis running buffer is in a range of, for example, 4.5 to 6. In the electrophoresis running buffer, the concentration of an anionic group-containing compound is in a range of, for example, 0.001 to 10 wt%.

An electrophoresis chip of the present invention may further include a pretreatment reservoir for hemolyzing and diluting a sample containing glycosylated hemoglobin, and the pretreatment reservoir and at least one fluid reservoir of the plurality of fluid reservoirs may be in communication with each other. It is preferable that the pretreatment reservoir is in communication with at least one fluid reservoir of the first introduction reservoir and the second introduction reservoir, and it is more preferable that the pretreatment reservoir is only in communication with either the first introduction reservoir or the second introduction reservoir.

Glucosylated hemoglobin to be analyzed with an electrophoresis chip of the present invention is not particularly limited, and examples include HbA1c, labile HbA1c, and GHbLys, among others, with HbA1c being particularly preferable.

An electrophoresis chip of the present invention may be configured such that:
the substrate includes an upper substrate and a lower substrate,
a plurality of through-holes are formed in the upper substrate,
a groove is formed in the lower substrate,
the upper substrate is laminated onto the lower substrate,
spaces created by sealing the bottom parts of the plurality of through-holes formed in the upper substrate with the lower substrate serve as the plurality of fluid reservoirs, and
a space created by sealing the upper part of the groove formed in the lower substrate with the upper substrate serves as a capillary channel.

An electrophoresis chip of the present invention may be configured such that:
a plurality of concave portions and a groove are formed in a substrate,
a surface of the substrate is sealed with a sealing material that has openings at places corresponding to the plurality of concave portions,
the plurality of concave portions formed in the substrate serve as a plurality of fluid reservoirs, and
a space created by sealing the upper part of the groove formed in the substrate with the sealing material serves as a capillary channel.

An electrophoresis chip of the present invention may be configured such that:
the electrophoresis chip further includes a sealing material,
a plurality of through-holes are formed in a substrate,
a groove is formed in s bottom surface of the substrate,
the bottom surface of the substrate is sealed with the sealing material, spaces created by sealing the bottom parts of the plurality of through-holes formed in the substrate with the sealing material serve as a plurality of fluid reservoirs; and
a space created by sealing the lower part of the groove formed in the bottom surface of the substrate with the sealing material serves as a capillary channel.

An electrophoresis chip of the present invention may be configured such that a plurality of fluid reservoirs are in communication with each other via a capillary tube that is a member independent of the substrate, and the capillary tube may serve as the capillary channel.

In an electrophoresis chip of the present invention, the volumes of the plurality of fluid reservoirs are not particularly limited, and are each in a range of, for example, 1 to 1000 mm³ and preferably in a range of 50 to 100 mm³.

An electrophoresis chip of the present invention may be configured such that the electrophoresis chip further includes a plurality of electrodes, and the plurality of electrodes may be disposed such that their first ends are placed in the plurality of fluid reservoirs.

Next, an electrophoresis chip of the present invention is described with reference to embodiments. The present invention, however, is not limited to the embodiments presented below.

### Embodiment 1

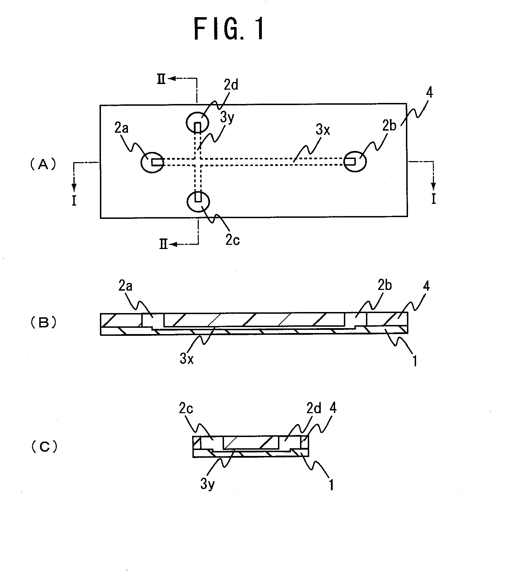

FIG. 1 shows an example of an electrophoresis chip of the present invention. FIG. 1(A) is a plan view of an electrophoresis chip of this embodiment, FIG. 1(B) is a cross-sectional view when taken along I-I of FIG. 1(A), and FIG. 1(C) is a cross-sectional view when taken along II-II of FIG. 1(A). For easier understanding, the size, proportions and like features of each component in the illustrations are different from the actual features of each component. This electrophoresis chip is, as shown in the figures, configured such that an upper substrate 4 is laminated onto a lower substrate 1. A plurality of through-holes (four in this embodiment) is formed in the upper substrate 4. The bottom parts of the four through-holes formed in the upper substrate 4 are sealed with the lower substrate 1 and, thus, fluid reservoirs 2a to 2d are formed. A cross-shaped groove is formed in the lower substrate 1. By sealing the upper part of the cross-shaped groove formed in the lower substrate 1 with the upper substrate 4, a capillary channel for sample analysis 3x and a capillary channel for sample introduction 3y are formed. The four fluid reservoirs 2a to 2d include a first introduction reservoir 2a, a first recovery reservoir 2b, a second introduction reservoir 2c and a second recovery reservoir 2d. The first introduction reservoir 2a and the first recovery reservoir 2b are in communication with each other via the capillary channel for sample analysis 3x. The first introduction reservoir 2c and the second recovery reservoir 2d are in communication with each other via the capillary channel for sample introduction 3y. The capillary channel for sample analysis 3x and the capillary channel for sample introduction 3y intersect. The capillary channel for sample analysis 3x and the capillary channel for sample introduction 3y are in communication with each other at the intersection. The electrophoresis chip of this embodiment is rectangular parallelepipedic. However, the present invention is not limited thereto. An electrophoresis chip of the present invention may be in any shape insofar as it does not adversely affect an electrophoresis measurement. The planar shape of an electrophoresis chip of this embodiment is rectangular. However, the present invention is not limited thereto. The planar shape of an electrophoresis chip of the present invention may be, for example, square or may be of another form. In an electrophoresis chip of this embodiment, the maximum length of the capillary channel for sample analysis 3x and the maximum length of the capillary channel for sample introduction 3y are different. However, the present invention is not limited thereto. In an electrophoresis chip of the present invention, the maximum length of the capillary channel for sample analysis 3x and the maximum length of the capillary channel for sample introduction 3y may be the same. Furthermore, an electrophoresis chip of this embodiment includes two capillary channels (3x, 3y). However, an electrophoresis chip of the present invention is not limited thereto. For example, an electrophoresis chip of the present invention may include a capillary channel for sample analysis 3x only. In this case, only the first introduction reservoir 2a and the first recovery reservoir 2b are formed in the lower substrate 1, and the first introduction reservoir 2a and the first recovery reservoir 2b are in communication with each other via the capillary channel for sample analysis 3x. Furthermore, an electrophoresis chip of this embodiment includes two substrate pieces (upper substrate 4 and lower substrate 1). However, an electrophoresis chip of the present invention is not limited thereto. An electrophoresis chip of the present invention may be composed of, for example, a single-piece substrate as described below.

Next, a method for producing an electrophoresis chip of this embodiment is described. An electrophoresis chip, however, may be produced according to methods other than the production method described below.

In an electrophoresis chip of this embodiment, a substrate formed from, for example, a glass material, a polymeric material or the like can be used as the lower substrate 1. Examples of a glass material include synthetic silica glass, and borosilicate glass, among others. Examples of a polymeric material include polymethylmethacrylate (PMMA), cycloolefin polymer (COP), polycarbonate (PC), polydimethylsiloxane (PDMS), polystyrene (PS), and polylactic acid, among others.

In an electrophoresis chip of this embodiment, the length and the width of the lower substrate 1 correspond to the maximum length and the maximum width of the whole chip described above, respectively. Therefore, the length and the width of the lower substrate 1 are arranged to be identical to the maximum length and the maximum width of the whole chip described above, respectively. The thickness of the lower substrate 1 in the electrophoresis chip of this embodiment is in a range of, for example, 0.1 to 3 mm and preferably in a range of 0.1 to 1 mm.

The material of the upper substrate 4 is not particularly limited insofar as it does not adversely affect an absorbance measurement that will be described below. For example, an upper substrate that is formed from the same material as the lower substrate 1 can be used as the upper substrate 4.

The length and the width of the upper substrate 4 are the same as the length and the width of the lower substrate 1, respectively. The thickness of the upper substrate 4 is suitably determined according to the volumes or like factors of the plurality of fluid reservoirs 2a to 2d and, for example, it is in a range of 0.1 to 3 mm and preferably in a range of 1 to 2 mm.

The width and the depth of the cross-shaped groove (the capillary channel for sample analysis 3x and the capillary channel for sample introduction 3y) are suitably determined according to the maximum diameter of the capillary channel and, for example, the width thereof is in a range of 25 to 200 µm and the depth thereof is in a range of 25 to 200 µm, and preferably the width thereof is in a range of 40 to 100 µm and the depth thereof is in a range of 40 to 100 µm. The maximum length of the capillary channel for sample analysis 3x and the maximum length of the capillary channel for sample introduction 3y are as described above.

The volumes of the plurality of fluid reservoirs 2a to 2d are as described above. In FIG. 1, the shapes of the plurality of fluid reservoirs 2a to 2d are cylindrical. However, an electrophoresis chip of the present invention is not limited to this embodiment. In an electrophoresis chip of the present invention, the shapes of the plurality of fluid reservoirs are not particularly limited insofar as the introduction and the recovery of a sample are not adversely affected, which will be described below and, for example, the reservoirs can be in any shape such as a quadrangular prism, a quadrangular pyramid, a cone or a combination of these shapes. Furthermore, the volumes and the shapes of the plurality of fluid reservoirs may all be the same or may each be different.

In an electrophoresis chip of this embodiment, the maximum thickness of the whole chip is the sum of the thickness of the lower substrate 1 and the thickness of the upper substrate 4. The maximum thickness of the whole chip is as described above.

For example, when the material of the lower substrate 1 is glass, the electrophoresis chip can be produced as follows.

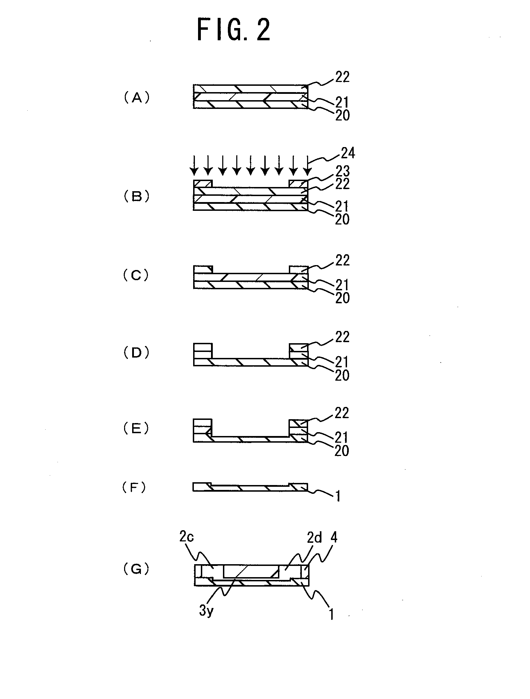

First, a surface of a glass plate 20 is masked with an alloy 21 of chromium and gold as shown in FIG. 2(A). A surface of the alloy 21 is then coated with a photoresist 22.

Next, a photosensitive film on which a layout pattern for the capillary channel for sample analysis 3x and the capillary channel for sample introduction 3y is drawn is adhered to a surface of the photoresist 22 as shown in FIG. 2(B) to prepare a photomask 23. Ultraviolet rays 24 are then irradiated over the photomask 23 for exposure.

Due to the exposure, the exposed portions of the photoresist 22 are solubilized as shown in FIG. 2(C) to form (transfer) the layout pattern on the alloy 21.

Next, the revealed portions of the alloy 21 are removed by aqua regia as shown in FIG. 2(D).

The layout pattern is then etched with hydrogen fluoride into the glass plate 20 as shown in FIG. 2(E).

Next, the photoresist 22 and the alloy 21 are removed to produce the lower substrate 1 as shown in FIG. 2(F).

Next, the upper substrate 4 is prepared (not shown). A method for forming the four through-holes in the upper substrate 4 is not particularly limited. For example, when the material of the upper substrate 4 is glass, an example of the formation method is ultrasonic machining or the like. For example, when the material of the upper substrate 4 is polymeric material, examples of the formation method include a cutting method; a molding method such as injection molding, cast molding and press molding using a metal mold; and like methods. The four through-holes may each be formed separately or may all be formed simultaneously. When the four through-holes are formed separately, they may be formed in any order. Forming all four through-holes simultaneously according to an aforementioned method that uses a metal mold or a like method requires a small number of steps and is thus preferable.

Finally, by laminating the lower substrate 1 and the upper substrate 4, an electrophoresis chip of this embodiment can be produced. A method for laminating the lower substrate 1 and the upper substrate 4 is not particularly limited and, for example, thermal welding is preferable. Although a production process was described in reference to FIG. 2, which shows cross sections corresponding to that shown in FIG. 1(C), the same production process can be applied to the cross section shown in FIG. 1(B).

For example, when the material of the lower substrate 1 is polymeric material, the electrophoresis chip can be produced as follows.

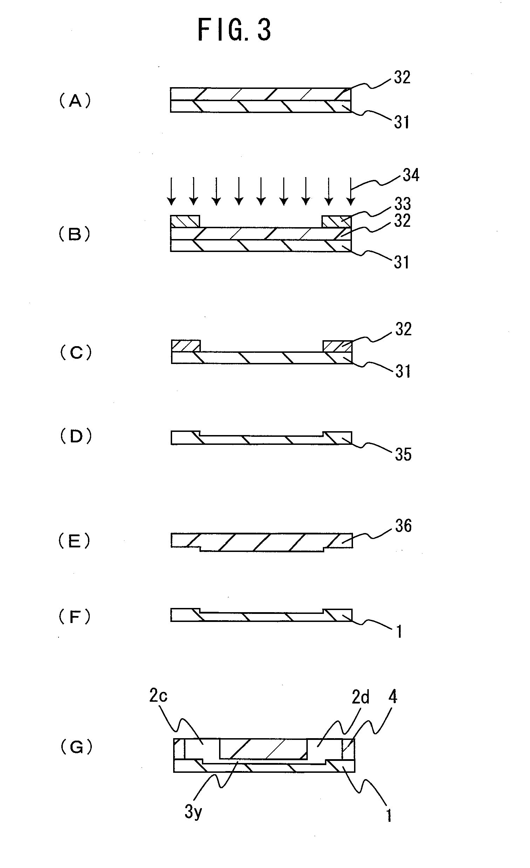

First, a surface of a silicon plate 31 is coated with a photoresist 32 as shown in FIG. 3(A).

Next, a photosensitive film on which a layout pattern for the capillary channel for sample analysis 3x and the capillary channel for sample introduction 3y is drawn is adhered to a surface of the photoresist 32 as shown in FIG. 3(B) to prepare a photomask 33. Ultraviolet rays 34 are then irradiated over the photomask 33 for exposure.

Due to the exposure, the exposed portions of the photoresist 32 are solubilized as shown in FIG. 3(C) to form (transfer) the layout pattern on the silicon plate 31.

Next, the layout pattern is etched into the silicon plate 31 to prepare a base mold 35 as shown in FIG. 3(D). Examples of etching include dry etching, and anisotropic etching, among others. The etching is preferably dry etching in view of the dimensional accuracy and the surface smoothness of the capillary channel for sample analysis 3x and the capillary channel for sample introduction 3y.

Metallic nickel electrocasting is then performed on the base mold 35 to prepare a metal mold for injection molding 36 as shown in FIG. 3(E).

Next, a lower substrate 1 composed of polymeric material is prepared by injection molding using the metal mold for injection molding 36 as shown in FIG. 3(F).

Next, the upper substrate 4 is prepared (not shown). A method for preparing the upper substrate 4 is the same as the method used when the material of the lower substrate 1 is glass.

Finally, by laminating the lower substrate 1 and the upper substrate 4, an electrophoresis chip of this embodiment can be produced. A method for laminating the lower substrate 1 and the upper substrate 4 is the same as the method used when the material of the lower substrate 1 is glass. Although a production process was described in reference to FIG. 3, which shows cross sections corresponding to that shown in FIG. 1(C), the same production process can be applied to the cross section shown in FIG. 1(B).

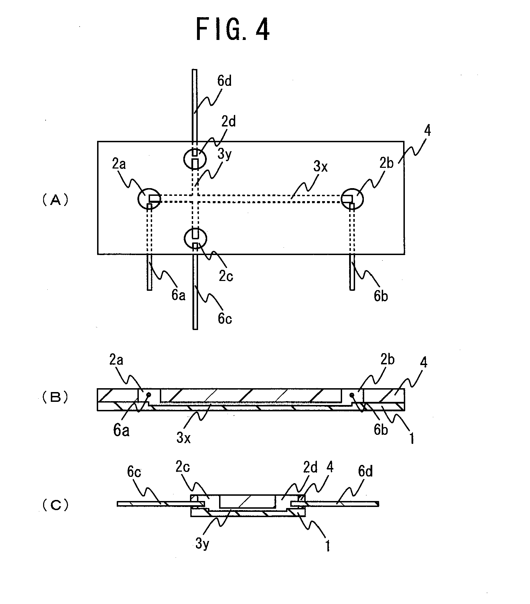

As described above, the electrophoresis chip of the present invention may further include a plurality of electrodes. FIG. 4 shows an electrophoresis chip of this embodiment that includes a plurality of electrodes. In FIG. 4, the portions that are identical to those in FIG. 1 are given the same numbers and symbols. As shown in FIG. 4, this electrophoresis chip has four electrodes 6a to 6d. The four electrodes 6a to 6d are disposed such that their first ends are placed in the plurality of fluid reservoirs 2a to 2d. The four electrodes 6a to 6d are embedded in the upper substrate 4. The four electrodes 6a to 6d can be readily disposed in position by creating, in advance, introduction holes for receiving the four electrodes 6a to 6d in side surfaces of the upper substrate 4, for example, when producing the upper substrate 4.
In an electrophoresis chip of the present invention, the plurality of electrodes is optional. The plurality of electrodes may be inserted into the plurality of fluid reservoirs, for example, when an electrophoresis chip is used.

The plurality of electrodes 6a to 6d may be any electrodes insofar as they are functional with an electrophoresis method. The plurality of electrodes 6a to 6d are each, for example, a stainless steel (SUS) electrode, a platinum (Pt) electrode, a gold (Au) electrode or the like.

An electrophoresis chip of the present invention may further include a pretreatment reservoir for hemolyzing and diluting a sample containing glycosylated hemoglobin. A hemolysis treatment for a sample is not particularly limited and, for example, it may be a treatment in which a sample is hemolyzed with a hemolytic agent. The hemolytic agent destroys, for example, the blood cell membrane of a blood cell component present in a sample that will be described below. Examples of hemolytic agents include the aforementioned electrophoresis running buffer, saponin, and "Triton X-100" (trade name) manufactured by Nacalai Tesque, Inc., among others, with the electrophoresis running buffer being particularly preferable. It is preferable that the pretreatment reservoir is in communication with, for example, an aforementioned introduction reservoir. The pretreatment reservoir may be formed in a suitable place such as a place near an aforementioned fluid reservoir with which the pretreatment reservoir is in communication such as, for example, the second introduction reservoir 2c. When a pretreatment reservoir is provided, a sample that will be described below is introduced into the pretreatment reservoir. The sample thus pretreated is introduced, via a channel that connects the pretreatment reservoir and an aforementioned fluid reservoir that is in communication with the pretreatment reservoir such as, for example, the second introduction reservoir 2c, into the second introduction reservoir 2c. The pretreatment reservoir may be configured such that two reservoirs, i.e., a reservoir for hemolyzing the sample and a reservoir for diluting the sample, are in communication.

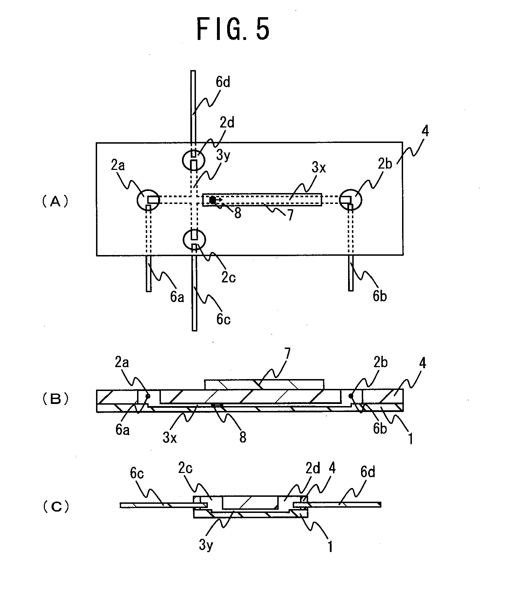

FIG. 5 shows an example of an electrophoresis apparatus that includes an electrophoresis chip of this embodiment. In FIG. 5, the portions that are identical to those in FIG. 1 and FIG. 4 are given the same numbers and symbols. As shown in FIG. 5, this electrophoresis apparatus includes an analysis unit 7. In an electrophoresis apparatus of this embodiment, the analysis unit 7 is a detector (line detector). The line detector is disposed on the upper substrate 4 such that the line detector is located over the capillary channel for sample analysis 3x on the first recovery reservoir 2b side relative to the intersection of the capillary channel for sample analysis 3x and the capillary channel for sample introduction 3y. A light source and a detection unit are housed in a line detector. The line detector emits light toward a sample from the light source and detects light reflected from the sample at the detection unit to measure absorbance. The analysis unit 7 is not limited to the line detector, and can be anything insofar as it can perform an analysis of glycosylated hemoglobin. The analysis unit 7 may be composed of, for example, a light source disposed under the electrophoresis chip and a detection unit disposed in a place corresponding to where the line detector is disposed. In this case, light is emitted from the light source toward a sample, and light transmitted by the sample is detected at the detection unit to measure absorbance.

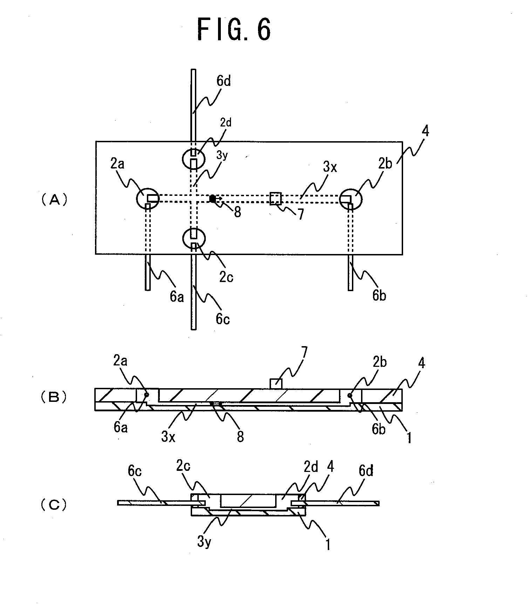

FIG. 6 shows another example of an electrophoresis apparatus that includes an electrophoresis chip of this embodiment. In FIG. 6, the portions that are identical to those in FIG. 5 are given the same numbers and symbols. As shown in FIG. 6, an electrophoresis apparatus of this embodiment has the same configuration as an electrophoresis apparatus shown in FIG. 5 except that the analysis unit 7 is different. As in this embodiment, the analysis unit 7 may measure absorbance at one point.

Next, a method for analyzing glycosylated hemoglobin in connection with the present invention is described using, as examples, the cases where the electrophoresis apparatuses shown in FIG. 5 and FIG. 6 are used.

First, the capillary channel for sample analysis 3x and the capillary channel for sample introduction 3y are filled with an electrophoresis running buffer by pressure or capillary action. The electrophoresis running buffer is as described above.

When the capillary channels are filled with an electrophoresis running buffer in advance, when the electrophoresis apparatus is not in use (when not in analysis), it is possible to omit the above-described step of filling with an electrophoresis running buffer and to immediately advance to the following steps, and it is thus preferable.

Next, a sample to be analyzed (a sample containing glycosylated hemoglobin) is introduced into the second introduction reservoir 2c. At this time, it is preferable to introduce a diluted sample that is diluted so as to have a volume ratio of the sample : the electrophoresis running buffer in a range of 1:4 to 1:99. That is, it is preferable that, in a method for analyzing glycosylated hemoglobin using an electrophoresis apparatus (electrophoresis chip) of the present invention, a diluted sample that is prepared by diluting a sample containing the glycosylated hemoglobin with an electrophoresis running buffer is introduced into at least one fluid reservoir of the plurality of fluid reservoirs, and the volume ratio of the sample : the electrophoresis running buffer is in a range of 1:4 to 1:99. However, the volume ratio is not limited to this. When an electrophoresis chip includes a pretreatment reservoir (not shown), the sample is introduced into the pretreatment reservoir and is pretreated therein. Next, a voltage is applied to the electrode 6c and the electrode 6d to generate a potential difference between both ends of the capillary channel for sample introduction 3y, thereby moving the sample to the intersection of the capillary channel for sample analysis 3x and the capillary channel for sample introduction 3y. The sample may be anything insofar as it contains hemoglobin (Hb), and examples include whole blood, hemolyzed samples prepared by subjecting whole blood to a hemolysis treatment, and like samples. Examples of hemolysis treatments include sonication treatment, freeze/thaw treatment, pressure treatment, osmotic pressure treatment, and surfactant treatment, among others. The hemolysis treatment may be performed in, for example, a pretreatment reservoir. Alternatively, a sample that has been subjected to a hemolysis treatment in advance in a separate apparatus or the like may be introduced into an electrophoresis apparatus (electrophoresis chip). The sample may be suitably diluted with, for example, water, physiological saline, or an electrophoresis running buffer, among others. This dilution may be performed in, for example, a pretreatment reservoir. Moreover, a sample that has been subjected to a dilution treatment in advance in a separate apparatus or the like may be introduced into an electrophoresis apparatus (electrophoresis chip).

The potential difference between the electrode 6c and the electrode 6d is in a range of, for example, 0.5 to 5 kV.

Next, a voltage is applied to the electrode 6a and the electrode 6b to generate a potential difference between both ends of the capillary channel for sample analysis 3x. In this manner, by instantly shifting a capillary channel having different potentials at both ends from the capillary channel for sample introduction 3y to the capillary channel for sample analysis 3x, the sample 8 is moved toward the first recovery reservoir 2b side from the intersection of the capillary channel for sample analysis 3x and the capillary channel for sample introduction 3y as indicated by the arrows in FIG. 5 and FIG 6.

The potential difference between the electrode 6a and the electrode 6b is in a range of, for example, 0.5 to 5 kV.

Next, each component of the sample that is separated due to the differences in migration speed is detected with the detector 7. It is thus possible to analyze (separate and measure) each component of the sample. According to the present invention, it is possible to analyze (separate and measure) with high accuracy glycosylated hemoglobin and other components of a sample that contains hemoglobin (Hb).

### Embodiment 2

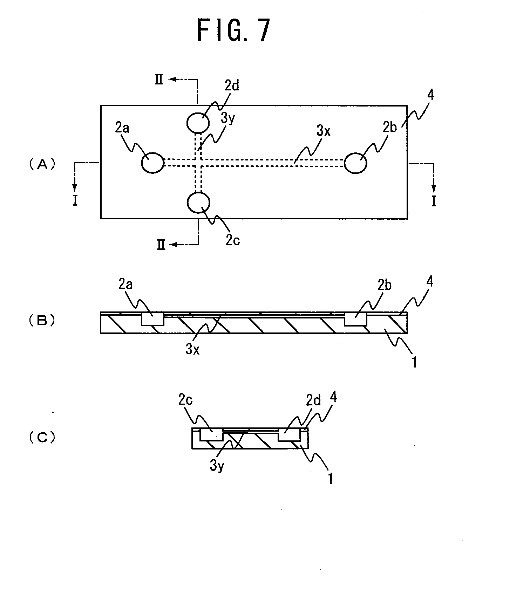

FIG. 7 shows another example of an electrophoresis chip of the present invention. In FIG. 7, the portions that are identical to those in FIG. 1 are given the same numbers and symbols. In an electrophoresis chip of this embodiment, a plurality of concave portions (four in this embodiment) and a cross-shaped groove are formed in a substrate (lower substrate) 1. A surface of the substrate (lower substrate) 1 is sealed with a sealing material (upper substrate) 4 that has openings at places corresponding to the four concave portions. The four concave portions formed in the substrate (lower substrate) 1 serve as four fluid reservoirs 2a to 2d. By sealing the upper part of the cross-shaped groove formed in the substrate (lower substrate) 1 with the sealing material (upper substrate) 4, a capillary channel for sample analysis 3x and a capillary channel for sample introduction 3y are formed. Otherwise an electrophoresis chip of this embodiment is of the same configuration as the electrophoresis chip shown in FIG. 1.

An electrophoresis chip of this embodiment can be produced, for example, as follows. However, the electrophoresis chip may be produced according to methods other than the production method described below.

For example, a substrate that is formed from the same material as the lower substrate 1 of the electrophoresis chip shown in FIG. 1 can be used as the substrate (lower substrate) 1.

In an electrophoresis chip of this embodiment, the length and the width of the substrate (lower substrate) 1 correspond to the maximum length and the maximum width of the whole chip described above, respectively. Therefore, the length and the width of the substrate (lower substrate) 1 are arranged to be identical to the maximum length and the maximum width of the whole chip described above, respectively. The thickness of the substrate (lower substrate) 1 in an electrophoresis chip of this embodiment is in a range of, for example, 0.1 to 3 mm and preferably in a range of 1 to 2 mm.

The material of the sealing material (upper substrate) 4 is also not particularly limited and, for example, a substrate that is formed from the same material as the lower substrate 1 of the electrophoresis chip shown in FIG. 1 can be used.

The length and the width of the sealing material (upper substrate) 4 are identical to the length and the width of the substrate (lower substrate) 1, respectively. The thickness of the sealing material (upper substrate) 4 is in a range of, for example, 50 to 1000 µm and preferably in a range of 100 to 300 µm.

For example, a commercially available sealing material may be used as the sealing material (upper substrate) 4 wherein holes are created in places corresponding to the four concave portions (the four fluid reservoirs 2a to 2d).

In an electrophoresis chip of this embodiment, the maximum thickness of the whole chip is the sum of the thickness of the substrate (lower substrate) 1 and the thickness of the sealing material (upper substrate) 4. The maximum thickness of the whole chip is as described above.

An example of a process for producing an electrophoresis chip of this embodiment is described below. However, an electrophoresis chip may be produced according to processes other than the production process described below.

First, the substrate (lower substrate) 1 is prepared. A method for forming the capillary channel for sample analysis 3x and the capillary channel for sample introduction 3y in the substrate (lower substrate) 1 is not particularly limited, and the capillary channels may be formed, for example, in the same manner as in Embodiment 1 above. A method for forming the four fluid reservoirs 2a to 2d in the substrate (lower substrate) 1 is also not particularly limited. For example, when the material of the substrate (lower substrate) 1 is glass, an example of the formation method is ultrasonic machining or the like. For example, when the material of the substrate (lower substrate) 1 is polymeric material, examples of the formation method include a cutting method; a molding method such as injection molding, cast molding and press molding using a metal mold; and like methods. The four fluid reservoirs 2a to 2d may each be formed separately or may all be formed simultaneously. When the four fluid reservoirs 2a to 2d are formed separately, they may be formed in any order. Forming all of the four through-holes simultaneously according to an aforementioned method that uses a metal mold or a like method requires a small number of steps and is thus preferable.

Next, by sealing a surface of the substrate (lower substrate) 1 with the sealing material (upper substrate) 4 in which holes are created in places corresponding to the four concave portions (the four fluid reservoirs 2a to 2d), an electrophoresis chip of this embodiment can be produced.

The configuration of an electrophoresis chip of this embodiment is not limited to that shown in FIG. 7. For example, as in FIG. 4 and other figures, a plurality of electrodes may be included, and the above-described pretreatment reservoir or the like may suitably be included. The configuration of an electrophoresis apparatus that uses the electrophoresis chip of this embodiment is also not particularly limited and, for example, a detector as in the electrophoresis apparatus of FIG. 5 or FIG. 6 may be included. Moreover, a method for analyzing glycosylated hemoglobin that uses an electrophoresis apparatus is also not particularly limited, and can be carried out, for example, in the same manner as with the case where the electrophoresis apparatus shown in FIG. 5 or FIG. 6 is used.

### Embodiment 3

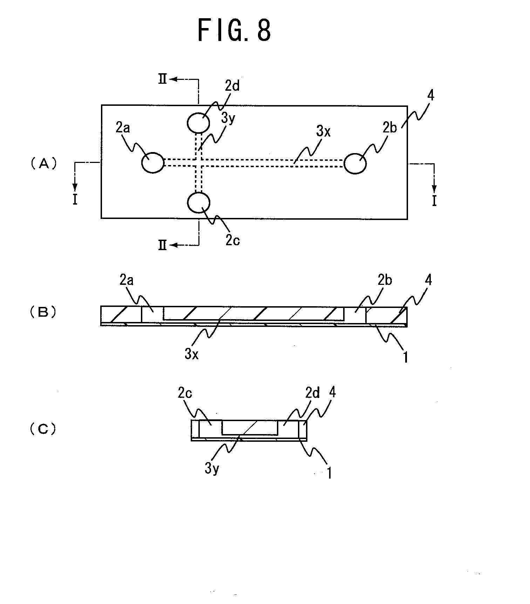

FIG. 8 shows still another example of an electrophoresis chip of the present invention. In FIG. 8, the portions that are identical to those in FIG. 1 are given the same numbers and symbols. In an electrophoresis chip of this embodiment, a plurality of through-holes (four in this embodiment) are formed in a substrate (upper substrate) 4. A cross-shaped groove is formed in the bottom surface of the substrate (upper substrate) 4. The bottom surface of the substrate (upper substrate) 4 is sealed with a sealing material (lower substrate) 1. The bottom parts of the four through-holes formed in the substrate (upper substrate) 4 are sealed with the sealing material (lower substrate) 1, and thereby four fluid reservoirs 2a to 2d are formed. By sealing the lower part of the cross-shaped groove formed in the substrate (upper substrate) with the sealing material, a capillary channel for sample analysis 3x and a capillary channel for sample introduction 3y are formed. Otherwise an electrophoresis chip of this embodiment is of the same configuration as an electrophoresis chip shown in FIG. 1.

An electrophoresis chip of this embodiment can be produced, for example, as follows. However, an electrophoresis chip may be produced according to methods other than the production method described below.

For example, a substrate that is formed from the same material as the lower substrate 1 of the electrophoresis chip shown in FIG. 1 can be used as the substrate (upper substrate) 4.

In an electrophoresis chip of this embodiment, the length and the width of the substrate (upper substrate) 4 correspond to the maximum length and the maximum width of the whole chip described above, respectively. Therefore, the length and the width of the substrate (upper substrate) 4 are arranged to be identical to the maximum length and the maximum width of the whole chip described above, respectively. The thickness of the substrate (upper substrate) 4 in the electrophoresis chip of this embodiment is in a range of, for example, 0.1 to 3 mm and preferably in a range of 1 to 2 mm.

The material of the sealing material (lower substrate) 1 is also not particularly limited and, for example, a substrate that is formed from the same material as the lower substrate 1 of the electrophoresis chip shown in FIG. 1 can be used.

The length and the width of the sealing material (lower substrate) 1 are identical to the length and the width of the substrate (upper substrate) 4, respectively. The thickness of the sealing material (upper substrate) 4 is in a range of, for example, 50 to 1000 µm and preferably in a range of 100 to 300 µm.

For example, a commercially available sealing material may be used for the sealing material (lower substrate) 1.

In an electrophoresis chip of this embodiment, the maximum thickness of the whole chip is the sum of the thickness of the substrate (upper substrate) 4 and the thickness of the sealing material (lower substrate) 1.
The maximum thickness of the whole chip is as described above.

An example of a process for producing an electrophoresis chip of this embodiment is described below. However, an electrophoresis chip may be produced according to processes other than the production process described below.

First, the substrate (upper substrate) 4 is prepared. A method for forming the capillary channel for sample analysis 3x and the capillary channel for sample introduction 3y in the substrate (upper substrate) 4 is not particularly limited, and the capillary channels may be formed, for example, in the same manner as in Embodiment 1 above. A method for forming the four through-holes in the substrate (upper substrate) 4 is also not particularly limited, and the through-holes may be formed, for example, in the same manner as in Embodiment 1 above.

Next, by sealing the bottom surface of the substrate (upper substrate) 4 with the sealing material (lower substrate) 1, an electrophoresis chip of this embodiment can be produced.

The configuration of an electrophoresis chip of this embodiment is not limited to that shown in FIG. 8. For example, as in FIG. 4 and other figures, a plurality of electrodes may be included, and the above-described pretreatment reservoir and the like may suitably be included. The configuration of an electrophoresis apparatus that uses the electrophoresis chip of this embodiment is also not particularly limited and, for example, a detector as in the electrophoresis apparatus of FIG. 5 or FIG. 6 may be included. Moreover, a method for analyzing glycosylated hemoglobin that uses an electrophoresis apparatus is also not particularly limited, and can be carried out, for example, in the same manner as with the case where the electrophoresis apparatus shown in FIG. 5 or FIG. 6 is used.

### Embodiment 4

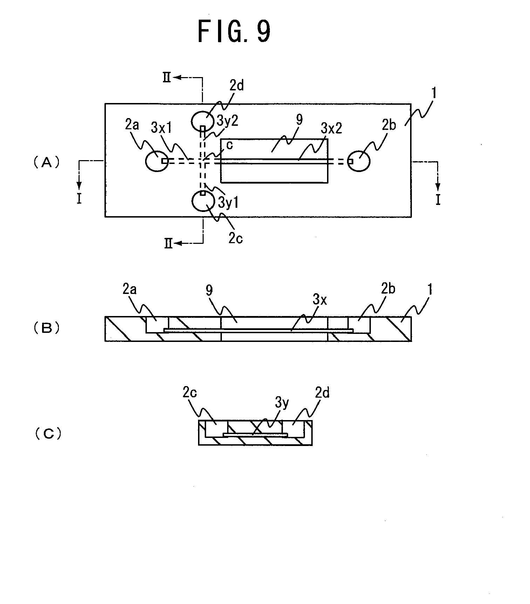

FIG. 9 shows still another example of an electrophoresis chip of the present invention. In FIG. 9, the portions that are identical to those in FIG. 1 are given the same numbers and symbols. An electrophoresis chip of this embodiment has a single-piece substrate, and the plurality of fluid reservoirs are in communication with each other via capillary tubes that are members independent of the substrate. The capillary tubes are composed of four capillary tubes 3x1, 3x2, 3y1 and 3y2. One end of each of the four capillary tubes gathers at the central portion c and connects with each other. As a result, the four capillary tubes are internally in communication with each other. The substrate 1 is provided with cavities (not shown) for the insertion of the four capillary tubes. The capillary tube 3x1 is inserted into substrate 1 such that the other end thereof is located on the bottom surface of the first introduction reservoir 2a. The capillary tube 3x2 is inserted into substrate 1 such that the other end thereof is located on the bottom surface of the first recovery reservoir 2b. The capillary tubes 3x1 and 3x2 serve as the capillary channel for sample analysis 3x. The capillary tube 3y1 is inserted into substrate 1 such that the other end thereof is located on the bottom surface of the second introduction reservoir 2c. The capillary tube 3y2 is inserted into substrate 1 such that the other end thereof is located on the bottom surface of the second recovery reservoir 2d. The capillary tubes 3y1 and 3y2 serve as the capillary channel for sample introduction 3y. The plurality of fluid reservoirs 2a to 2d are each formed as a concave portion in substrate 1. Substrate 1 has a rectangular parallelepipedic opening (window) 9 on the first recovery reservoir 2b side relative to the capillary channel for sample introduction 3y. Otherwise an electrophoresis chip of this embodiment is of the same configuration as an electrophoresis chip shown in FIG. 1.

An electrophoresis chip of this embodiment can be produced, for example, as follows. However, an electrophoresis chip may be produced according to methods other than the production method described below.

For example, a substrate that is formed from the same material as lower substrate 1 of the electrophoresis chip shown in FIG. 1 can be used as the substrate 1.

In an electrophoresis chip of this embodiment, the length, the width and the thickness of substrate 1 correspond to the maximum length, the maximum width and the maximum thickness of the whole chip described above, respectively. Therefore, the length, the width and the thickness of substrate 1 are arranged to be identical to the maximum length, the maximum width and the thickness of the whole chip described above, respectively.

The inner diameter of each of the four capillary tubes is the same as the maximum diameter of the above-described capillary channel. The length of each of the four capillary tubes is determined according to the maximum length of the capillary channel for sample analysis 3x and the maximum length of the capillary channel for sample introduction 3y.

An example of a process for producing an electrophoresis chip of this embodiment is described below. However, an electrophoresis chip may be produced according to processes other than the production process described below.

First, substrate 1 is prepared. A method for forming the four fluid reservoirs 2a to 2d and the opening (window) 9 in substrate 1 is not particularly limited and, for example, the fluid reservoirs can be formed according to the same method as used for the four fluid reservoirs 2a to 2d of an electrophoresis chip shown in FIG. 7. The fluid reservoirs 2a to 2d and the opening (window) 9 may each be formed separately or may all be formed simultaneously. When the four fluid reservoirs 2a to 2d and the opening (window) 9 are formed separately, they may be formed in any order.
Forming all of the four fluid reservoirs 2a to 2d and the opening (window) 9 simultaneously according to an aforementioned method that uses a metal mold or a like method requires a small number of steps and is thus preferable.

Next, the four capillary tubes are inserted into substrate 1. In this manner, the electrophoresis chip of this embodiment can be obtained.

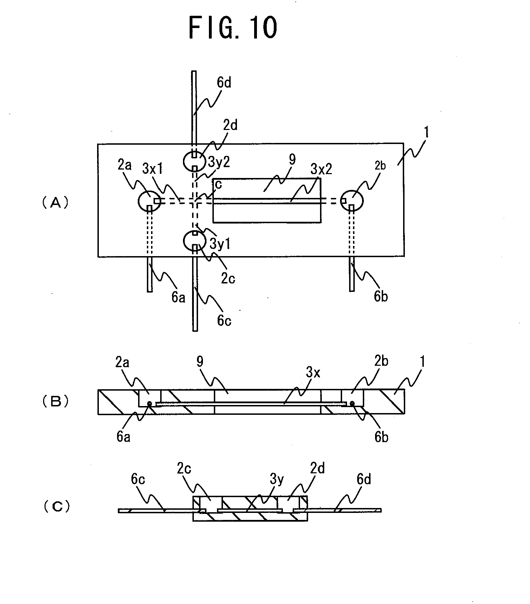

FIG. 10 shows an electrophoresis chip of this embodiment that has a plurality of electrodes. In FIG. 10, the portions that are identical to those in FIG. 4 are given the same numbers and symbols. As shown in FIG. 10, four electrodes 6a to 6d are embedded in substrate 1 in this electrophoresis chip. Otherwise an electrophoresis chip of this embodiment is of the same configuration as an electrophoresis chip shown in FIG. 4. The four electrodes 6a to 6d can be readily disposed in position by creating, in advance, introduction holes for receiving the four electrodes 6a to 6d in side surfaces of substrate 1, for example, when producing substrate 1.

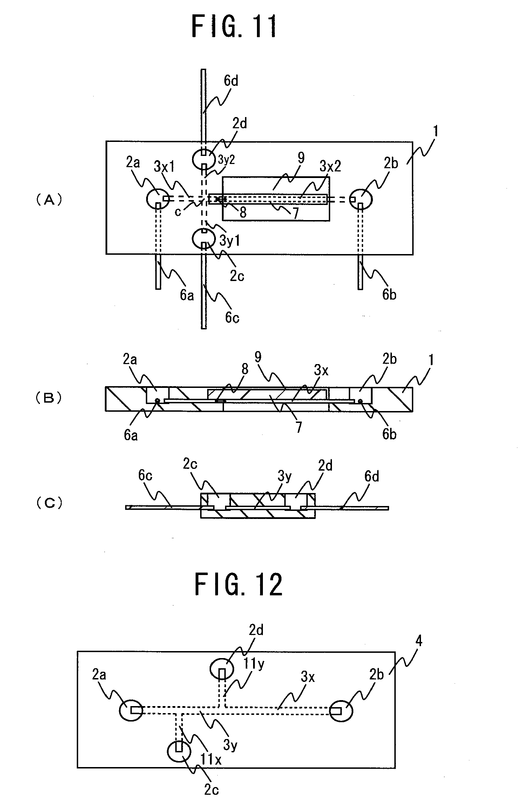

FIG. 11 shows an example of an electrophoresis apparatus that includes an electrophoresis chip of this embodiment. In FIG. 11, the portions that are identical to those in FIG. 5 are given the same numbers and symbols. As shown in FIG. 11, an analysis unit (line detector) 7 is directly disposed on an aforementioned capillary tube in this electrophoresis apparatus. Moreover, in this electrophoresis apparatus, substrate 1 is provided with, in addition to the cavities into which the four capillary tubes are to be inserted, a cavity into which an analysis unit (line detector) 7 is to be inserted (not shown). Otherwise, an electrophoresis apparatus of this embodiment has the same configuration as the electrophoresis apparatus shown in FIG. 5. An electrophoresis apparatus of this embodiment is not limited by the configuration shown in FIG. 11 and, for example, a detector as in the electrophoresis apparatus of FIG. 6 may be included. An analysis of glycosylated hemoglobin using the electrophoresis apparatus of this embodiment can be carried out in the same manner as in the case where an electrophoresis apparatus shown in FIG. 5 or FIG. 6 is used.

### Embodiment 5

FIG. 12 shows still another example of an electrophoresis chip of the present invention. In FIG. 12, the portions that are identical to those in FIG. 1 are given the same numbers and symbols. FIG. 12 is a plan view of an electrophoresis chip of this embodiment. As shown in FIG. 12, in this electrophoresis chip, a groove having a shape of two "T"s combined is formed in place of a cross-shaped groove in the lower substrate 1 (not shown) and, thereby, a capillary channel for sample analysis 3x and a capillary channel for sample introduction 3y are formed. That is, first, the capillary channel for sample analysis 3x is linear, and the first introduction reservoir 2a and the first recovery reservoir 2b are in communication with each other via the capillary channel for sample analysis 3x. A first branching channel 11x branches off from a part of the capillary channel for sample analysis 3x. The first branching channel 11x is in communication with the second introduction reservoir 2c. A second branching channel 11y branches off from a part of the capillary channel for sample analysis 3x that is located on the downstream side (right-hand side on FIG. 12) relative to the first branching channel 11x. The second branching channel 11y is in communication with the second recovery reservoir 2d. The capillary channel for sample introduction 3y is formed by the first branching channel 11x, the second branching channel 11y and the part of the capillary channel for sample analysis 3x that connects the branching channels. The first branching channel 11x and the second branching channel 11y are substantially perpendicular to the capillary channel for sample analysis 3x and form together with the capillary channel for sample analysis 3x a groove having a shape of two "T"s combined. Otherwise an electrophoresis chip of this embodiment is of the same configuration as an electrophoresis chip shown in FIG. 1.

The configuration of an electrophoresis chip of this embodiment is not limited to the configuration shown in FIG. 12. For example, an electrophoresis chip may be composed of a single-piece substrate as shown in FIG. 8. Moreover, an electrophoresis chip may be provided with a plurality of electrodes as shown in FIG. 4 and FIG. 10 and may be suitably provided with a pretreatment reservoir as described above. A method for producing an electrophoresis chip of this embodiment is also not particularly limited, and may be identical to, for example, the production methods described in Embodiments 1 to 4 above. The configuration of an electrophoresis apparatus that uses an electrophoresis chip of this embodiment is also not particularly limited and, for example, a detector as in the electrophoresis apparatus of FIG. 5, FIG. 6 or FIG. 11 may be provided therein. Moreover, a method for analyzing glycosylated hemoglobin that uses an electrophoresis apparatus is also not particularly limited, and can be carried out, for example, in the same manner as in the case where an electrophoresis apparatus shown in FIG. 5, FIG. 6 or FIG. 11 is used. Example

An analysis of HbA1c was carried out using an electrophoresis apparatus shown in FIG. 5. That is, first, the capillary channel for sample analysis 3x and the capillary channel for sample introduction 3y were filled with an electrophoresis running buffer by applying pressure. A buffer prepared by adding chondroitin C in a proportion of 0.5 wt% to a solution of 100 mM malic acid that had been adjusted to pH 5.5 by addition of arginine was used as the electrophoresis running buffer.

Next, a sample was introduced into the second introduction reservoir 2c. An Hb control sample was used as the sample. Next, a voltage of 0.60 kV was applied to the electrode 6c and no voltage was applied to the electrode 6d, thereby creating a potential difference between both ends of the capillary channel for sample introduction 3y. The sample was thereby moved to the intersection of the capillary channel for sample analysis 3x and the capillary channel for sample introduction 3y. In this case, a voltage of 0.30 kV was applied to both electrode 6a and electrode 6b.

Next, while a voltage of 0.40 kV was applied to both the electrode 6c and the electrode 6d, a voltage of 1.00 kV was applied to the electrode 6a and no voltage was applied to the electrode 6b, thereby creating a potential difference between both ends of the capillary channel for sample analysis 3x. The sample 8 was thereby moved from the intersection of the capillary channel for sample analysis 3x and the capillary channel for sample introduction 3y toward the first recovery reservoir 2b side.

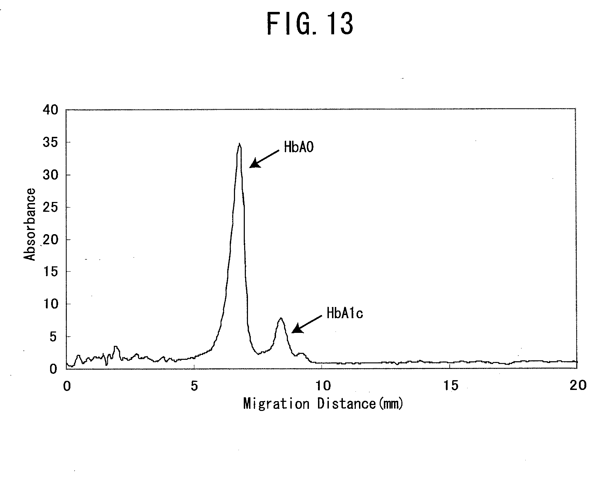

Next, the relationship between the absorbance and the distance (migration distance) from the intersection of the capillary channel for sample analysis 3x and the capillary channel for sample introduction 3y was measured with the line detector 7. The results of the measurement are shown in the graph of FIG. 13. As shown in FIG. 13, in this example, it was possible to detect HbA1c separately from other components such as HbA0 present in the sample. The time (migration time) required for analysis was very short at 20 seconds.

### Industrial Applicability

An electrophoresis chip of the present invention enables an apparatus to be small, analysis time to be short, and glycosylated hemoglobin to be analyzed with high accuracy. An electrophoresis chip of the present invention is applicable to all technical fields where glycosylated hemoglobin is analyzed, such as laboratory tests, biochemical examinations and medical research. The intended use of the electrophoresis chip is not limited and it is applicable to a broad range of technical fields.

## Claims

1. An electrophoresis chip for analyzing glycosylated hemoglobin comprising:
a substrate, a plurality of fluid reservoirs and a capillary channel,
the plurality of fluid reservoirs comprises a first introduction reservoir and a first recovery reservoir,
the capillary channel comprises a capillary channel for sample analysis,
the first introduction reservoir and the first recovery reservoir being formed in the substrate, and
the first introduction reservoir and the first recovery reservoir being in communication with each other via the capillary channel for sample analysis.

2. The electrophoresis chip according to claim 1, wherein
the plurality of fluid reservoirs further comprises a second introduction reservoir and a second recovery reservoir,
the capillary channel further comprises a capillary channel for sample introduction,
the second introduction reservoir and the second recovery reservoir are formed in the substrate,
the second introduction reservoir and the second recovery reservoir are in communication with each other via the capillary channel for sample introduction,
the capillary channel for sample introduction and the capillary channel for sample analysis intersect, and
the capillary channel for sample introduction and the capillary channel for sample analysis are in communication with each other at the intersection.

3. The electrophoresis chip according to claim 2, wherein
a first branching channel branches off from a part of the capillary channel for sample analysis,
the first branching channel is in communication with the second introduction reservoir,
a second branching channel branches off from a part of the capillary channel for sample analysis that is located on the downstream side relative to the first branching channel,
the second branching channel is in communication with the second recovery reservoir, and
the capillary channel for sample introduction is formed by the first branching channel, the second branching channel and a part of the capillary channel for sample analysis that connects the branching channels.

4. The electrophoresis chip according to claim 1, having an maximum length of the whole chip in a range of 10 to 100 mm, a maximum width of the whole chip in a range of 10 to 60 mm, and a maximum thickness of the whole chip in a range of 0.3 to 5 mm.

5. The electrophoresis chip according to claim 1, wherein, in analyzing the glycosylated hemoglobin, a diluted sample prepared by diluting a sample containing the glycosylated hemoglobin with an electrophoresis running buffer is introduced into at least one fluid reservoir of the plurality of fluid reservoirs, and a volume ratio of the sample : the electrophoresis running buffer is 1:4 to 1:99.

6. The electrophoresis chip according to claim 1, wherein the capillary channel is filled with an electrophoresis running buffer.

7. The electrophoresis chip according to claim 1, wherein the capillary channel has a maximum diameter in a range of 10 to 200 µm and a maximum length of 0.5 to 15 cm.

8. The electrophoresis chip according to claim 1, further comprising a pretreatment reservoir for hemolyzing and diluting a sample containing the glycosylated hemoglobin, the pretreatment reservoir and at least one fluid reservoir of the plurality of fluid reservoirs being in communication with each other.

9. The electrophoresis chip according to claim 1, wherein the glycosylated hemoglobin is HbA1c.

10. The electrophoresis chip according to claim 1, wherein
the substrate comprises an upper substrate and a lower substrate,
a plurality of through-holes are formed in the upper substrate,
a groove is formed in the lower substrate,
the upper substrate is laminated onto the lower substrate,
spaces created by sealing the bottom parts of the plurality of through-holes formed in the upper substrate with the lower substrate serve as the plurality of fluid reservoirs, and
a space created by sealing the upper part of the groove formed in the lower substrate with the upper substrate serves as the capillary channel.

11. The electrophoresis chip according to claim 1, wherein
a plurality of concave portions and a groove are formed in the substrate,
a surface of the substrate is sealed with a sealing material that has openings at places corresponding to the plurality of concave portions,
the plurality of concave portions formed in the substrate serve as the plurality of fluid reservoirs, and
a space created by sealing the upper part of the groove formed in the substrate with the sealing material serves as the capillary channel.

12. The electrophoresis chip according to claim 1, wherein
a sealing material is further included,
a plurality of through-holes are formed in the substrate,
a groove is formed in the bottom surface of the substrate,
the bottom surface of the substrate is sealed with the sealing material,
spaces created by sealing the bottom parts of the plurality of through-holes formed in the substrate with the sealing material serve as the plurality of fluid reservoirs, and
a space created by sealing the lower part of the groove formed in the bottom surface of the substrate with the sealing material serves as the capillary channel.

13. The electrophoresis chip according to claim 1, wherein the plurality of fluid reservoirs are in communication with each other via a capillary tube that is a member independent of the substrate, and the capillary tube serves as the capillary channel.

14. The electrophoresis chip according to claim 1, wherein the plurality of fluid reservoirs each has a volume in a range of 1 to 1000 mm³.

15. The electrophoresis chip according to claim 1, further comprising a plurality of electrodes,
wherein the plurality of electrodes are disposed such that their one ends are placed respectively in the plurality of fluid reservoirs.

16. An electrophoresis apparatus comprising an electrophoresis chip and an analysis unit,
wherein the electrophoresis chip being the electrophoresis chip according to claim 1.
